# EUROPEAN PATENT SPECIFICATION

(11) **EP 0 507 854 B1**
(45) Date of publication and mention of the grant of the patent: **27.11.1996**
(21) Application number: 91902414.1
(22) Date of filing: 27.12.1990
(51) Int. Cl.: C12Q 1/34, C07H 17/075, C07H 17/02, C07H 15/203, C07H 17/00

(54) **METHODS FOR DIAGNOSING HUMAN INFLUENZA AND 4-POSITION MODIFIED CHROMOGENIC N-ACETYLNEURAMINIC ACID SUBSTRATES FOR USE THEREIN**
VERFAHREN ZUR DIAGNOSTIZIERUNG VON MENSCHLICHER GRIPPE UND IN POSITION 4 MODIFIZIERTE CHROMOGENE N-ACETYLNEURAMINSÄURE SUBSTRATE ZUR VERWENDUNG IN DIESEN VERFAHREN
METHODE DE DIAGNOSTIC DE LA GRIPPE CHEZ L'HOMME ET SUBSTRATS D'ACIDE N-ACETYLNEURAMINIQUE CHROMOGENE MODIFIE EN POSITION 4 UTILISES DANS LESDITES METHODES

(30) Priority: 29.12.1989 US 458805
(43) Date of publication of application: 14.10.1992
(73) Proprietor: SYMEX CORP., Broken Arrow, OK 74012 (US)
(72) Inventor: TURNER, Gregory, A., Broken Arrow, OK 74012 (US); MAHER, James, F., Broken Arrow, OK 74012 (US); CLINKSCALES, C., Worth, Tulsa, OK 74133 (US); ROARK, Michael, C., Owasso, OK 74055 (US)
(74) Representative: Goldin, Douglas Michael
(86) International application number: US9007681
(87) International publication number: WO9109972

(56) References cited:
- HELVETICA CHIMICA ACTA, vol. 69, no. 8, 1986, Basel (CH); F. BAUMBERGER et al., pp. 1927-1935; and H.-W. HAGEDORN et al., pp. 2127-2133/
- HELVETICA CHIMICA ACTA, vol. 69, no. 7, 1986, Basel (CH); F. BAUMBERGER et al., pp. 1545-1541
- CHEMICAL ABSTRACTS, vol. 93, no. 5, 04 August 1980, Columbus, OH (US); J.M. BEAU et al., p. 394, no. 40335/
- CHEMICAL ABSTRACTS, vol. 108, no. 1, 04 January 1988, Columbus, OH (US); H.J. GROSS et al., p. 250, no. 2531/
- JOURNAL OF INFECTIOUS DISEASES, vol. 142, no. 4, 1980; YOLKEN et al., pp. 516- 523/
- BIOLOGICAL ABSTRACTS, vol. 82, no. 12, 1987; TAKEI et al., no. 117445/
- BIOLOGICAL ABSTRACTS, vol. 81, no. 8, 1986; KIYOTANI et al., no. 78292/
- BIOLOGICAL ABSTRACTS, vol. 79, no. 3, 1985; KIYOTANI et al., no. 27461/

## Description

### Technical Field

The present invention relates to diagnostic tests for human influenza. More specifically it relates to chromogenic substrates that are useful in the diagnosis of influenza through the detection of the enzymatic activity of human influenza neuraminidase (NA).

### Background of the Invention

Influenza virus averages 30-50 million infections annually in the United States alone. Epidemiologic studies of influenza epidemics estimate the incidence of infection to be 25% in the general population and higher in school age children. Researchers have estimated that up to half the infected persons would see a physician because of the illness. In 1986, the Center for Disease Control (CDC) estimated that influenza epidemics have been associated with 10,000 or more excess deaths in 18 of the preceding 28 years. CDC studies indicate influenza as the fifth leading cause of death in the United States. Antigenic variations in the surface glycoproteins of influenza A and B account for their continued epidemics.

Influenza viruses possess surface glycoproteins that have NA activity. These glycoproteins are members of a family of neuraminidases that are found in viruses, bacteria, mycoplasmas, and animal tissues. They hydrolyze substrates that contain alpha-ketosidically linked N-acetylneuraminic acid (Neu5Ac; referred to previously as "NANA"). In viruses, NA typically constitutes 5-10% of the viral protein and exists as a mushroom-shaped spike on the envelope. Viral NA is composed of a hydrophilic area which includes the catalytic site of the enzyme and a hydrophobic area that is inserted into the viral envelope anchoring the enzyme to the virus.

Various assays for NA activity are described in the literature. Santer, U.V, et al., Biochimica et Biophysica Acta 523:435-442 (1978), describes a colorimetric assay for NA using 2-(3-methoxyphenyl)-N-acetyl-alpha-D neuraminic acid as a substrate and 4-aminoantipyrine in the presence of an oxidizing agent to measure the enzymatically released methoxyphenol. Myers, R.W.; et al., Analytical Biochemistry 101:166-174 (1980), describes the use of the 4-methyl- umbelliferylalpha-ketoside of Neu5Ac in a fluorometric assay for NA. This chromogenic derivative of Neu5Ac was also used in studies of the NA activity of influenza viruses by Yolken, R. H., et al., J. Infectious Diseases 142:516-523 (1980); Clinical Chemistry 27:1490-1498 (1981); and Reviews of Infectious Diseases 4:35-68 (1982); and by Kiyotani et al., Hiroshima J. Medical Sciences 33:287-292 (1984); Zbl Bakt Hyg A260-273-285 (1985); Microbiol. Immun. 31: 1131-1135 (1987). Despite the availability of these prior NA assays, however, physicians currently still diagnose influenza solely on the basis of symptomology This is in part due to the fact that these prior assays were complicated and/ or required equipment not typically found in a clinical setting. Another shortcoming of these prior assays is that they were unable to discriminate between influenza type. That ability is particularly important to enable physicians to prescribe the appropriate chemotherapy and/or supportive therapy to combat the infection.

Prior workers have investigated the relationship between the chemical structure of Neu5Ac and its biological function as a substrate for non-influenza NA. Gross, H.J., et al., Biochemistry 27:4279 (1988), examined benzyl-alpha- glycosides of N-acetyl-4-epi-D-neuraminic acid as a substrate for three different bacterial NAs (C. perfrinaens, A. ureafaciens, and V. cholera) and found significant differences in reactivity. After 22 hrs, the C. perfrinqens NA cleaved 100% of the substrate while the A. ureafaciens and V cholera NAs cleaved only 50% and 11 % of the substrate, respectively. Kim et al., J. Am. Chem. Soc. 110:6481-6486 (1988) described the structural characteristics of substrates accepted by Neu5Ac aldolase, its use in the synthesis of Neu5Ac, and its chemical conversion to the 2-deoxy derivatives, and additionally reported that work was in progress to determine the biological activity of the 2-deoxy derivatives. Brossmer et al., Helv. Chim. Acta 69:2127 (1986); Glycoconjugates 4:145 (1987) reported that the methyl-alpha-glycoside of 4-deoxy Neu5Ac was a good substrate for fowl plague viral Neu5Ac, but not for the three bacterial NAs mentioned above. Additionally, Schauer, R., et al., Eur. J. Biochem. 106:531 (1980), reported that 4-methoxy Neu5Ac was an excellent substrate for fowl plague viral NA but not for V. cholera NA. The 4-methylumbelliferyl derivative of 4-deoxy Neu5Ac is also described in the literature (Helv. Chim. Acta. 69:1927 (1986)). Zbiral et al., Monatsheft fur Chemie 119:127-141 (1988) described the synthesis of 7- and 8-deoxy Neu5Ac and 4,7-dideoxy Neu5Ac. Zbiral et al., Liebigs Ann Chem 119:127-141 (1989) described the synthesis of the 4-methylυmbelliferyl-2-a glycosides of 7-epi, 8-epi, 7,8-bis-epi, 8-deoxy, 9-deoxy and 4,7-dideoxy Neu5Ac and investigated the behavior of those compounds as inhibitors of the sialidase from V. cholera.

Applicant is unaware of any prior reports on the reactivity of 4-modified Neu5Acs with human influenza NA.

### Disclosure of the Invention

One aspect of the invention is a method of detecting human influenza neuraminidase activity in a clinical sample suspected of having such activity comprising:
(a) incubating the sample with a chromogenic modified N-acetylneuraminic acid substrate of the formula: 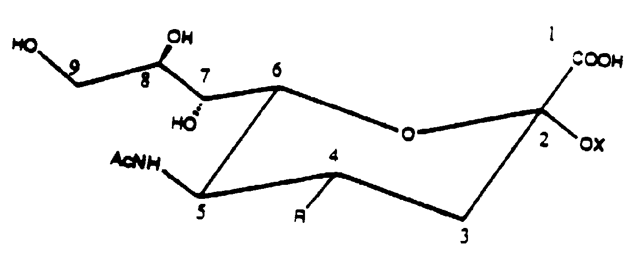 where Ac represents acetyl, R represents hydrogen, fluorine or methoxy, and X represents a chromogenic group that exhibits distinct color when cleaved from the substrate or a salt of said substrate; and
(b) detecting neuraminidase activity by observing whether the sample-substrate mixture exhibits said color after step (a).

Another aspect of the invention is a method of selectively detecting a specific type (e.g., AorB) of human influenza neuraminidase activity in a clinical sample suspected of having human influenza neuraminidase activity from activity exhibited by other types of human influenza neuraminidase comprising:
(a) incubating the sample with a chromogenic modified N-acetylneuraminic acid substrate of the formula: 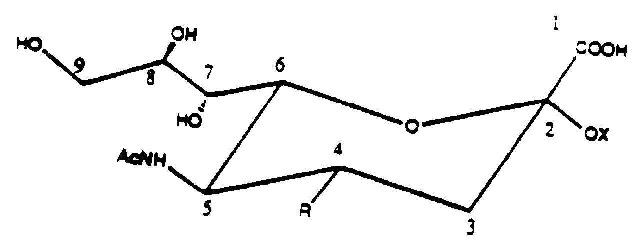 where Ac represents acetyl, R represents hydrogen, fluorine or methoxy, and X represents a chromogenic group that exhibits distinct color when cleaved from the substrate or a salt of said substrate;
(b) observing the color exhibited by the sample-substrate mixture after step (a); and
(c) comparing said color to colors exhibited by activity standards of human influenza neuraminidase of said specific type and other types of human influenza neuraminidase on said substrate.

Yet another aspect of the invention is a modified Neu5Ac chromogenic substrate useful for detecting human influenza neuraminidase activity in a clinical sample suspected of having such activity said substrate having the formula: 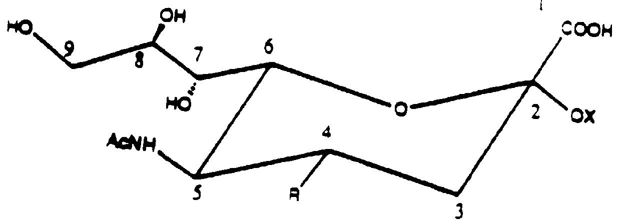 where Ac represents acetyl, R is methoxy or fluorine, and X is a chromogenic group that exhibits a distinct color when cleaved from the substrate and salts of said substrate.

Still another aspect of the invention is a chromogenic substrate useful for detecting human influenza neuraminidase activity in a clinical sample suspected of having such activity, said substrate having the formula: 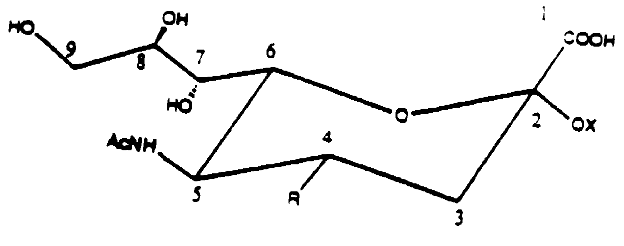 where Ac represents acetyl, R is hydrogen and X is a chromogenic group selected from the group consisting of 3-cyanoumbelliferyl, 2- or 4-nitrophenyl, 3-resorufin, 5-bromo-4-chloro-3-indolyl, 4-nitrophenylazophenyl 4-nitrophenylazoresorcinyl, 3-methoxyphenyl, 3-dimethylaminophenyl, 4-chloro-1-naphthyl, and 6-bromo-2-naphthyl and salts of said substrate.

### Brief Description of the Drawinqs

In the drawings:
Figure 1 is a schematic diagram depicting the synthesis procedure described in Example 1.
Figure 2 is a schematic diagram depicting the synthesis procedure described in Example 2.
Figure 3 is a schematic diagram depicting the synthesis procedure described in Example 3.
Figure 4 is a schematic diagram depicting the synthesis procedure described in Example 4.
Figure 5 is a schematic diagram depicting the synthesis procedure described in Example 5.
Figure 6 is a schematic diagram depicting the synthesis procedure described in Example 6.

### Modes for Carrying Out the Invention

The chromogenic modified N-acetylneuraminic acid substrates of the invention and the methods employing them are useful for detecting human influenza neuraminidase activity in clinical samples or specimens and for determining the type of human influenza neuraminidase present in the sample. Accordingly, these substrates and methods are useful for diagnosing influenza infection generally as well as the type of influenza infection present in the human patient from whom the clinical sample was collected. In this regard, the term "influenza" is intended to include influenza types A and B and parainfluenza types 1, 2, and 3. The term "selectively detect" intends the ability to detect NA activity of one type of influenza virus as compared to the activity of other types of influenza virus.

The clinical samples that are tested in the invention will typically be pharyngeal, nasopharyngeal or respiratory secretions collected from patients suffering from influenza as wash, swab, or expectorate specimens The wash, expectorate, or swab will preferably be combined with an aqueous buffer solution containing a stabilizer prior to mixing with the substrate. The buffer solution contains a buffer that maintains the pH at about 4 to 7, preferably 5.5 to 6.5, optionally about 0.1% to 10% by weight nonionic detergent, a small amount (1-20 mM) of alkaline earth metal cation (Ca, Mg, preferably Ca), and a sufficient amount of a stabilizer selected from the group consisting of alditols, simple sugars, and disaccharide sugars to enhance the thermal stability of the NA in the sample. The volume of buffer solution combined with the specimen will normally be 0.1 to 2 ml.

The buffer may be organic or inorganic. Examples of suitable buffers are conventional buffers of organic acids and salts thereof such as citrate buffers (e.g. monosodium citrate-disodium citrate mixture, citric acid-trisodium citrate mixture, citric acid-monosodium citrate mixture, etc.), acetate buffers (e.g., acetic acid-sodium acetate mixture), succinate buffers (e.g. succinic acid-monosodium succinate mixture, succinic acid-sodium hydroxide mixture, succinic acid-disodium succinate mixture, etc.), tartrate buffers (e.g. tartaric acid-tartrate mixture, tartaric acid-potassium tartrate mixture, tartaric acid-sodium hydroxide mixture etc.), fumarate buffers (e.g. fumaric acid-monosodium fumarate mixture, fumaric acid-disodium fumarate mixture, monosodium fumarate acid-disodium fumarate mixture), gluconate buffers (e.g. gluconic acid-sodium gluconate mixture, gluconic acid-sodium hydroxide mixture, gluconic acid-potassium gluconate mixture, etc.) oxalate buffers (e.g. oxalic acid-sodium oxalate mixture, oxalic acid-sodium hydroxide mixture, oxalic acid-potassium oxalate mixture, etc.), lactate buffers (e.g. lactic acid-sodium lactate mixture, lactic acid-sodium hydroxide mixture, lactic acid-potassium lactate mixture, etc.), acetate buffers (e.g. acetic acid-sodium acetate mixture, acetic acid-sodium hydroxide mixture, etc.), malate buffers (e.g., D,L-malic acid-disodium malate mixture), phosphate buffers (e.g. monosodium phosphate-disodium phosphate mixture, monosodium phosphate-sodium hydroxide mixture, trisodium phosphate-hydrochloric acid mixture, etc.), 2-(N-morpholino)ethanesulfonic acid, [bis-(2-hydroxyethyl)imino] tris(hydroxymethyl)methane, N-2-acetamidoiminodiacetic acid, 1,3-bis[tris(hydroxymethyl)methylamino]propane, piperazine-N,N'-bis(2-ethanesuifonic acid), N-2-acetamido-2-aminoethanesulfonic acid, 3-(N-morpholino)-2-hydroxypropanesulfonic acid, N-N-bis-(2-hydroxyethyl)2-aminoethanesulfonic acid, 3-(N-morpholino)propanesulfonic acid, 2-[tris (hydroxymethyl)methylamino]ethanesulfonic acid, N-2-hydroxyethylpiperazine-N'-2-ethanesulfonic acid, 3-{(tris-(hydroxymethyl)methyl]amino}-2-hydroxypropanesυlfonic acid.

Examples of non-ionic detergents useful in the buffer solution are the Pluronics, for example, Polysorbate 20 or Polysorbate 80, Triton@ X-100 NP-40, and alkyl glucosides such as C₈-C₉ alkyl glucoside. The detergent is an optional component and facilitates release of the NA from the viral envelope. "Triton" is a Trade Mark.

Examples of the stabilizers that are used in the buffer solution are trihydric or higher alditols, such as glycerin, erythritol, arabitol, xylitol, sorbitol, mannitol, the simple sugars glucose and fructose and the disaccharride sucrose. These polyhydric sugar alcohols, and simple and disaccharride sugars can be used alone or in combination. In order to stabilize the activity of the neuraminidase-containing viruses, the alditols or simple sugars and disaccharrides are added to the liquid formulation/excipient system in an amount from 0.2 M to 2.1 M and preferably, 0.6 M to 2.0 M.

Once mixed with the buffer solution, the sample may be stored for prolonged periods, preferably at 2°C to 8°C without significant loss of NA activity.

The substrate that is combined with the buffered, stabilized specimen is a chromogenic Neu5Ac derivative that is modified in the 4 position by removal of the hydroxyl group at that position, by replacement of the hydroxyl group with fluorine, or by replacement of the hydrogen of the hydroxyl group with a lower alkyl group. These substrates may be represented by the following chemical formula: 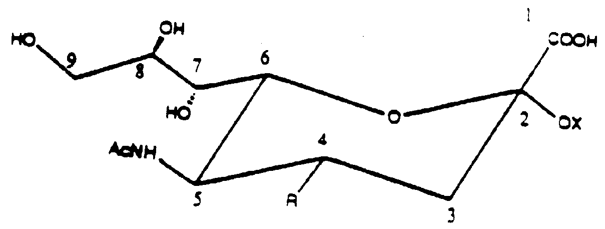 where R, X and Ac are as defined previously. Preferably X represents 4-methylumbelliferyl, 3-cyanoumbelliferyl, 2-nitrophenyl, 4-nitrophenyl, 3-resorufin, 5-bromo-4-chloro-3-indolyl, nitrophenylazophenyl, nitrophenylazoresorcinyl, 3-methoxyphenyl, 3-dimethylaminophenyl, 4-chloro-1-naphthyl, or 6-bromo-2-naphthyl. Simple salts of these substrates, such as the Na, K and NH₄⁺ salts, may also be used.

As used herein the term "chromogen" is intended to include, without limitation, molecules that exhibit fluorescence. The term "color" is likewise intended to include, without limitation, fluorescence.

Examples of 4-modified chromogenic Neu5Ac derivatives falling within the above formula are 4-methylumbelliferyl-4-deoxy-N-acetylneuraminic acid-alpha-ketoside, 3-cyanoumbelliferyl-4-deoxy-N-acetylneuraminic acid-alpha-ketoside, 2-nitrophenyl-4-deoxy-N-acetylneuraminic acid-alpha-ketoside, 4-nitrophenyl-4-deoxy-N-acetylneuraminic acid-alpha-ketoside, 3-resorufin-4-deoxy-N-acetylneuraminic acid-alpha-ketoside, 5-bromo-4-chloro-3-indolyl-4-deoxy-N-acetylneuraminic acid-alpha-ketoside, 2-[4-(4-nitrophenylazo)phenyl]-4-deoxy-N-acetylneuraminic acid-alpha-ketoside, 2-[4-(4-nitrophenylazo)resorcinyl]-4-deoxy-N-acetyl-neuraminic acid-alpha-ketoside, 3-methoxyphenyl-4-deoxy-N-acetylneuraminic acid-alpha-ketoside, 3-dimethylaminophenyl-4-deoxy-N-acetylneuraminic acid-alpha-ketoside, 4-chloro-1-naphthyl-4-deoxy-N-acetylneuraminic acid-alpha-ketoside, 6-bromo-2-naphthyl-4-deoxy-N-acetylneuraminic acid-alpha-ketoside, 4-methylumbelliferyl-4-methoxy-N-acetylneuraminic acid-alpha-ketoside, 2-nitrophenyl-4-methoxy-N-acetylneuraminic acid-alpha-ketoside, 4-nitrophenyl-4-methoxyN-acetylneuraminic acid-alpha-ketoside, 3-cyanoumbelliferyl-4-methoxy-N-acetylneuraminic acid-alpha-ketoside, 3-resorufin-4-methoxy-N-acetylneuraminic acid-alpha-ketoside, 5-bromo-4-chloro-3-indolyl-4-methoxy-N-acetylneuraminic acid-alpha-ketoside, 2-[4-(4-nitrophenylazo)phenyl]-4-methoxy-N-acetyl-neuraminic acid-alpha-ketoside, 2-[4-(4-nitrophenylazo)resorcinyl]-4-methoxy-N-acetylneuraminic acid-alpha-ketoside, 3-methoxyphenyl-4-methoxy-N-acetylneuraminic acid-alpha-ketoside, 3-dimethylaminophenyl-4-methoxy-N-acetylneuraminic acid-alpha-ketoside, 6-bromo-2-naphthyl-4-methoxy-N-acetylneuraminic acid-alpha-ketoside, 4-chloro-1-naphthyl-4-methoxy-N-acetylneuraminic acid-alpha-ketoside, 4-methylumbelliferyl-4-fluoro-N-acetylneuraminic acid-alpha-ketoside, 2-nitrophenyl-4-fluoro-N-acetylneuraminic acid-alpha-ketoside, 4-nitrophenyl-4-fluoro-N-acetylneuraminic acid-alpha-ketoside, 3-cyanoumbelliferyl-4-fluoro-N-acetylneuraminic acid-alpha-ketoside, 3-resorufin-4-methoxy-N-acetylneuraminic acid-alpha-ketoside, 5-bromo-4-chloro-3-indolyl-4-fluoro-N-acetylneuraminic acid-alpha-ketoside, 2-[4-(4-nitrophenylazo)phonyl]-4-fluoro-N-acetylneuraminic acid-alpha-ketoside, 2-[4-(4-nitrophenylazo)resorcinyl]-4-fluoro-N-acetylneuraminic acid-alpha-ketoside, 3-methoxyphenyl-4-fluoro-N-acetylneuraminic acid-alpha-ketoside, 3-dimethylaminophenyl-4-fluoro-N-acetylneuraminic acid-alpha-ketoside, 4-chloro-1-naphthyl-4-fluoro-N-acetylneuraminic acid-alpha-ketoside, and 6-bromo-2-naphthyl-4-fluoro-N-acetylneuraminic acid-alpha-ketoside.

The above-described Neu5Ac derivatives are generally made by protecting the functional groups of Neu5Ac at the 1, 2, 7, 8, and 9 positions, modifying the 4 position as indicated, deprotecting the 1, 2, 7, 8 and 9 positions, and coupling the 4-modified Neu5Ac with the chromogen Details of these reactions are provided in the Examples, infra.

The substrate will normally be added to the buffered, stabilized sample in amounts ranging between 0.05 mM and 0.5 mM. The mixture is incubated at ambient temperature to physiological temperature (i.e., about 22°C to 37°C) for a time sufficient to permit any NA in the sample to react with the substrate That time will normally be in the range of 20 to 120 minutes, more usually 30 to 60 minutes. If there is NA activity in the sample, the chromogenic group will be cleaved from the substrate and the liberated chromogen will impart a characteristic color to the mixture. Since the substrates of the invention may exhibit different reactivity to the different human influenza NAs, the specific type of influenza infection may be determined by comparing the color of the sample mixture with the color of standard reaction mixtures for each influenza NA type. For instance, influenza A may be distinguished from influenza B on the basis of substrate reactivity with the NAs of these influenza viruses. The following table indicates the color generated when NA reacts with a modified Neu5Ac and releases the chromogen.

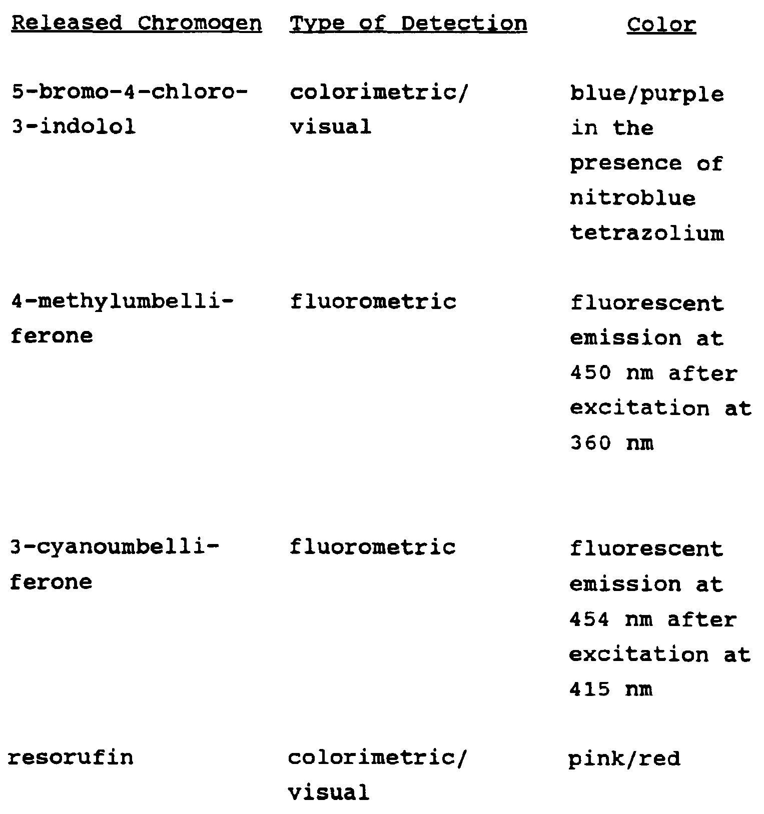

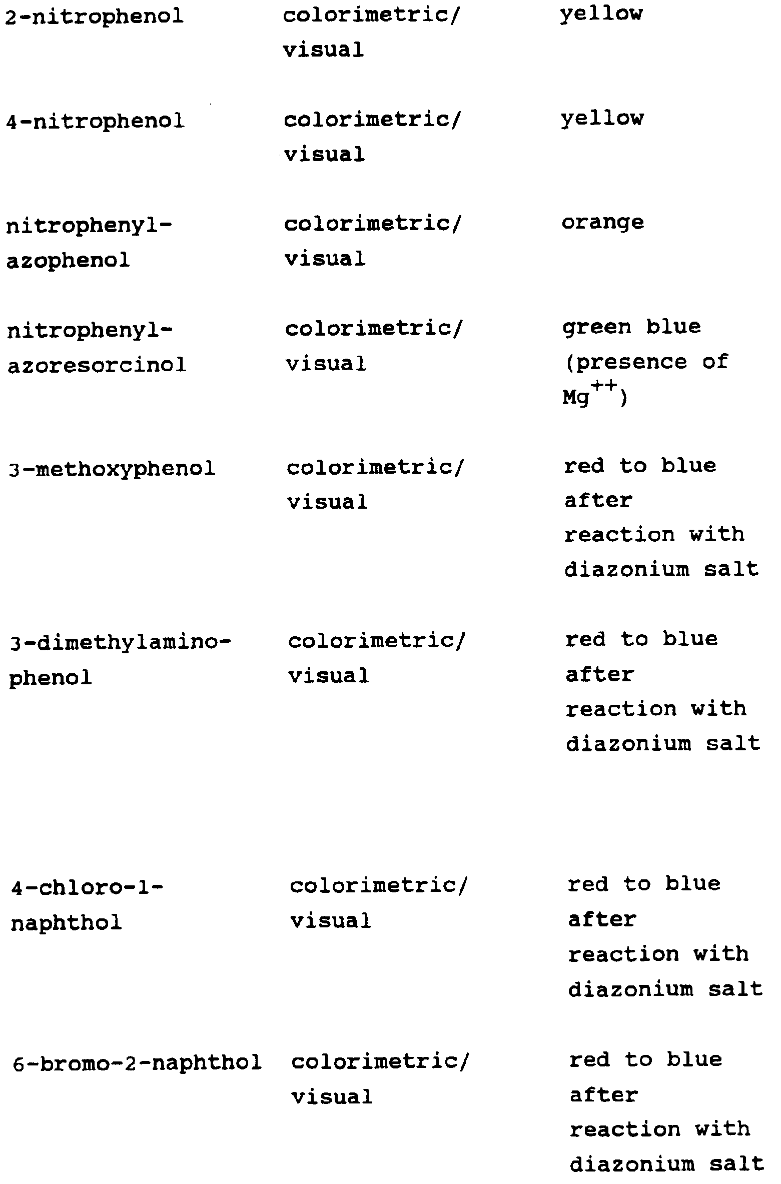

Accordingly, the present invention provides a simple and rapid technique for selectively diagnosing influenza that may be carried out in the clinic or physician's office and enable the physician to prescribe the appropriate therapy to treat the infection and/or the appropriate prophylactic treatment to persons in close contact with the infected patient.

The invention is further illustrated by the following examples. These examples are not intended to limit the invention in any manner.

### Examples

### 1. Synthesis of 4-Deoxy Neu5Ac

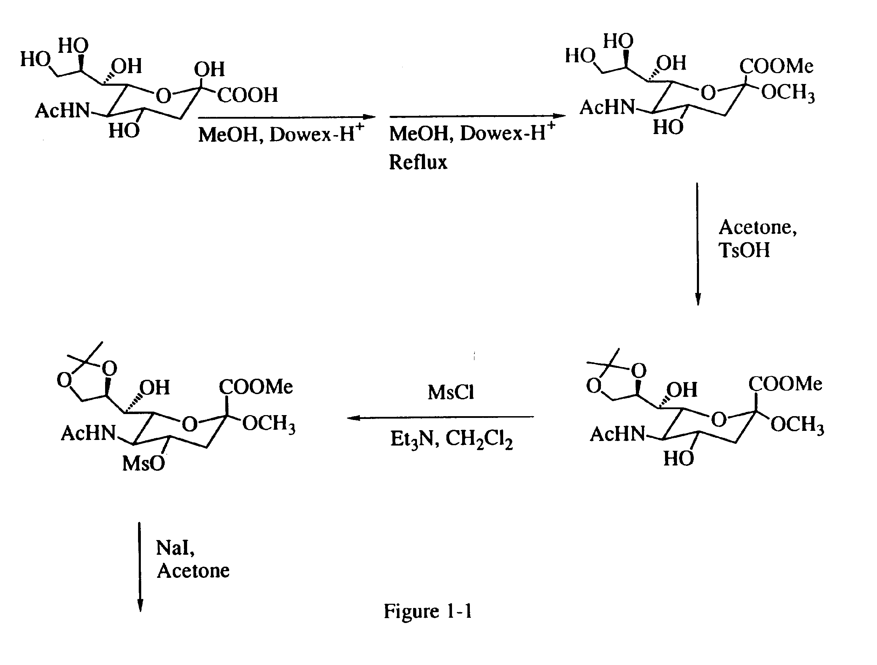

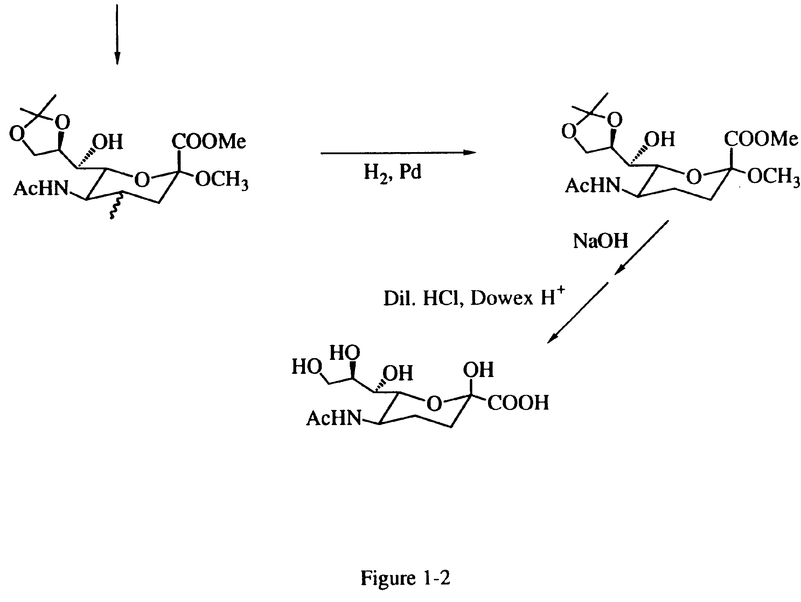

The reaction scheme for this synthesis is shown in Figure 1.

Neu5Ac (500 mg) in 100 ml of methanol and 1.25 g of Dowex®-50W (H⁺) are heated under reflux for 20 hr., and treated with decolorizing carbon to afford the methyl ester methyl ketoside of Neu5Ac (Neu5Ac-MEMK). "Dowex" is a Trade Mark. To the ketoside in dry acetone (dried over molecular sieves) is added catalytic p-toluenesulfonic acid monohydrate and the reaction allowed to stir for 4 hr. at room temperature (RT). Neutralization with Dowex®-1 (acetate form) and solvent evaporation gives 8,9-isopropylidene Neu5Ac methyl ester methyl ketoside. The 4-mesylate-8,9-isopropylidene Neu5Ac methyl ester methyl ketoside is obtained by treating with methanesulfonyl-chloride in methylene chloride and 1.5 equivalents of triethylamine at 0°C for 30 min. The mesylate is then treated with sodium iodide in acetone or methyl ethyl ketone and heated to ca. 100°C in a sealed reaction vial to form the 4-iodo compound. Subsequent hydrogenation over palladium on carbon affords the 4-deoxy molecule. Deprotection is accomplished by treatment first with sodium hydroxide followed by treatment with dilute HCI/Dowex@-50W (H⁺) to give 4-deoxy-N-acetylneuraminic acid.

### 2. Synthesis of 2-(p-Nilrophenyl)-4-Deoxy Neu5Ac

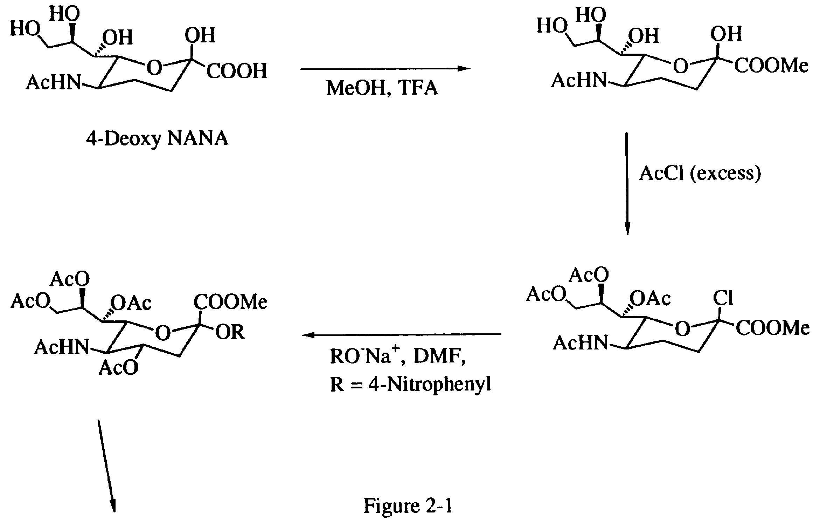

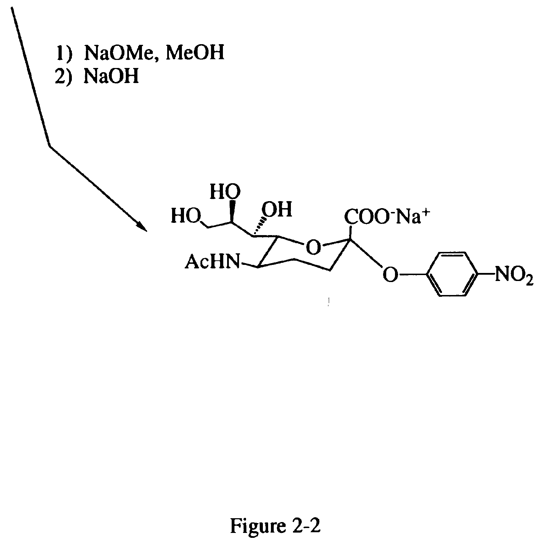

The reaction scheme for this synthesis is shown in Figure 2.

The 4-deoxy NeuSAc of Example 1 is converted to the corresponding methyl ester by stirring at room temperature (RT) with trifluoroacetic acid in methanol The methyl ester is then treated with an excess of acetyl chloride overnight to form the fully acetylated 2-chloro-4-deoxy Neu5Ac methyl ester. This intermediate is then treated with the sodium salt of the para-nitrophenol in dimethyl formamide (DMF) at RT for 2 hr. The coupled chromogenic 4-deoxy Neu5Ac is then deprotected by treatment with sodium methoxide in methanol (1 hr) followed by treatment with sodium hydroxide (2 hr) to form the sodium salt of 2-(p-nitrophenyl)-4-deoxy-N-acetylneuraminic acid.

### 3. Synthesis of 4-Methoxy Neu5Ac

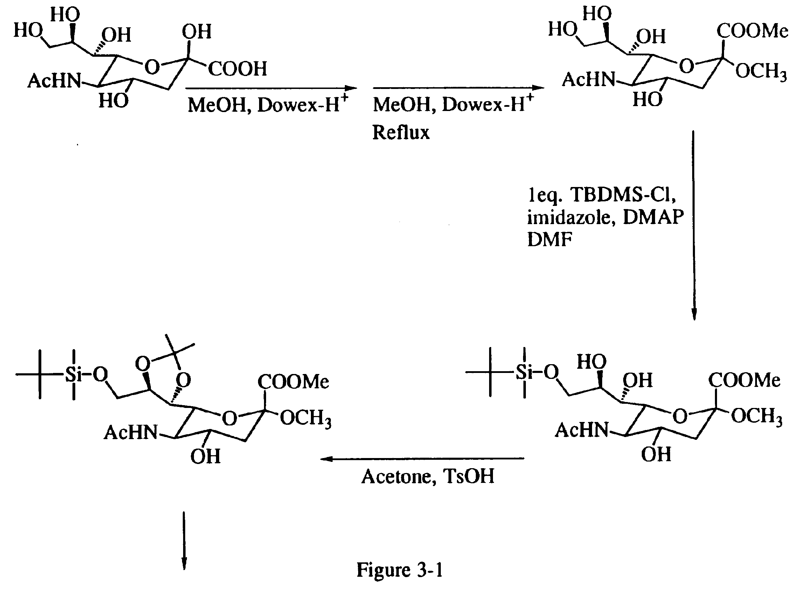

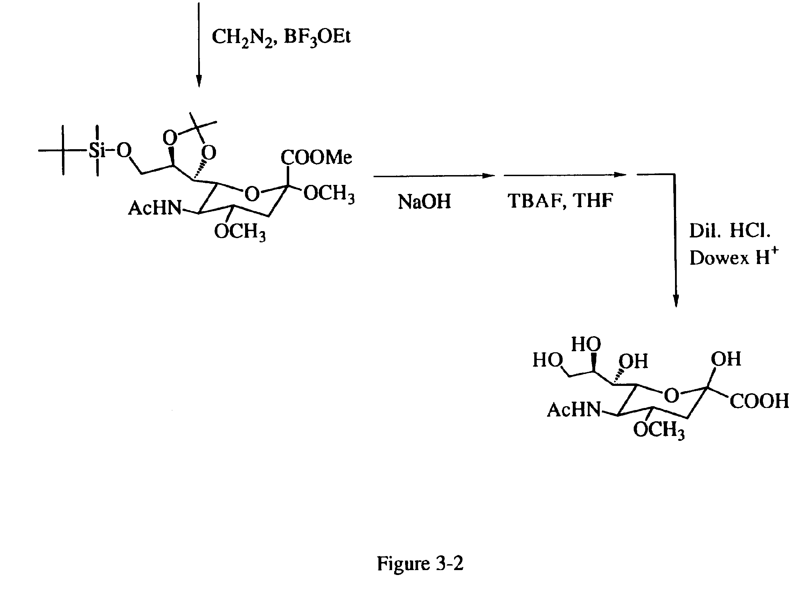

The reaction scheme for this synthesis is shown in Figure 3.

Neu5Ac-MEMK is treated with 1 eq. of tert-butyldimethylsilyl (TBDMS) chloride, imidazole and a catalytic amount of 4-dimethylaminopyridine at 65 C to afford 9-O-TBDMS Neu5Ac-MEMK. Treatment of this compound with acetone and a catalytic amount of p-toluenesulfonic acid monohydrate at RT yields 9-O-TBDMS-7,8-isopropylidene Neu5Ac-MEMK. Treatment with diazomethane/trifluoroborate in ether at 0°C gives the corresponding 4-methoxy derivative. This compound is fully deprotected by treatment with sodium hydroxide followed by tetrabutylammonium fluoride in THF and finally by treatment with dilute HCl/Dowex®-50W (H⁺) to give 4-methoxy-N-acetylneuraminic acid.

### 4. Synthesis of 2-(p-Nitrophenyl)-4-Methoxy Neu5Ac

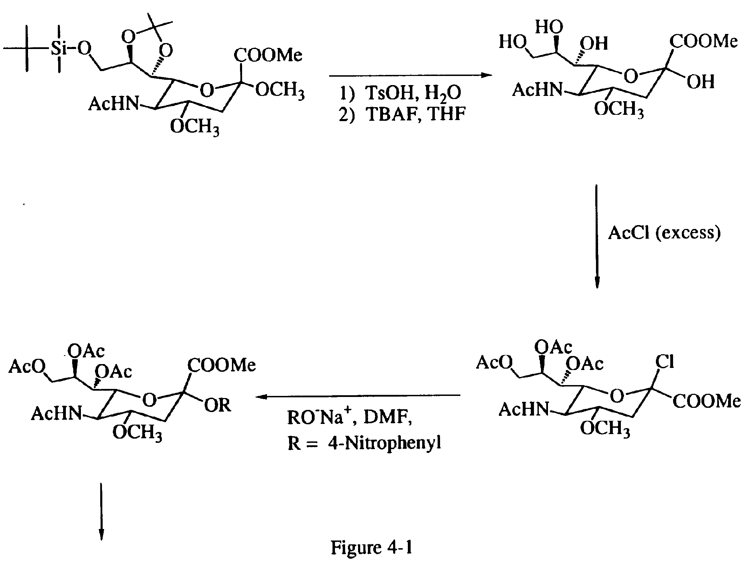

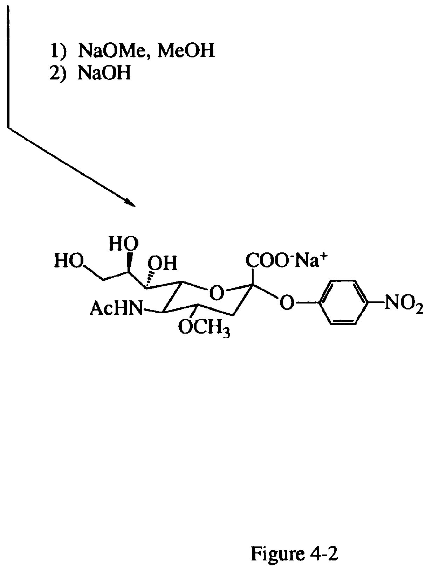

The reaction scheme for this synthesis is shown in Figure 4.

The 9-TBDMS-7,8-isopropylidene-protected 4-methoxy Neu5Ac-MEMK is deprotected with tosic acid followed by tetrabutylammonium fluoride in tetrahydrofuran. This intermediate is then treated with excess acetyl chloride overnight to form fully acetylated 2-chloro-4-methoxy Neu5Ac methyl ester. Coupling with the sodium salt of nitrophenol chromogen is done in dry DMF at RTfor2 2 hr. Deprotection and deprotonation is effected by treatment with sodium methoxide in methanol followed by treatment with sodium hydroxide to give 2-(p-nitrophenyl)-4-methoxy-N-acetylneuraminic acid, sodium salt.

### 5. Synthesis of 4-Fluoro Neu5Ac

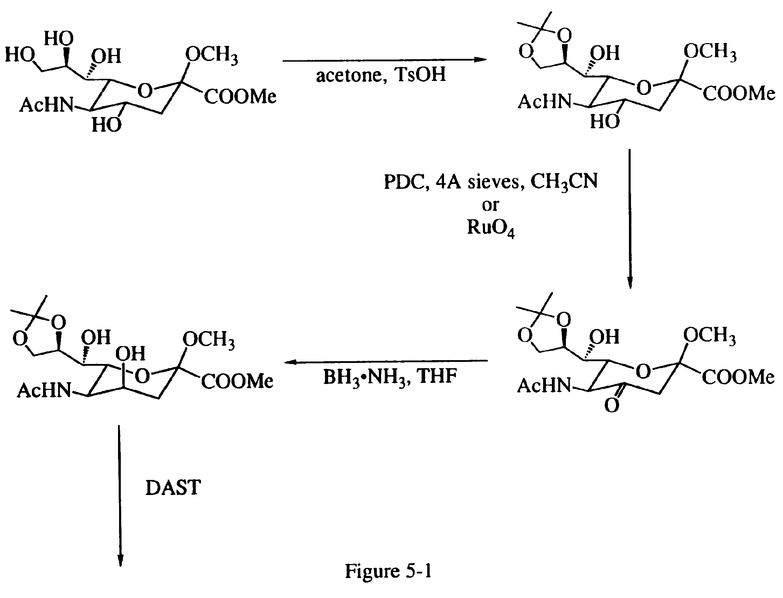

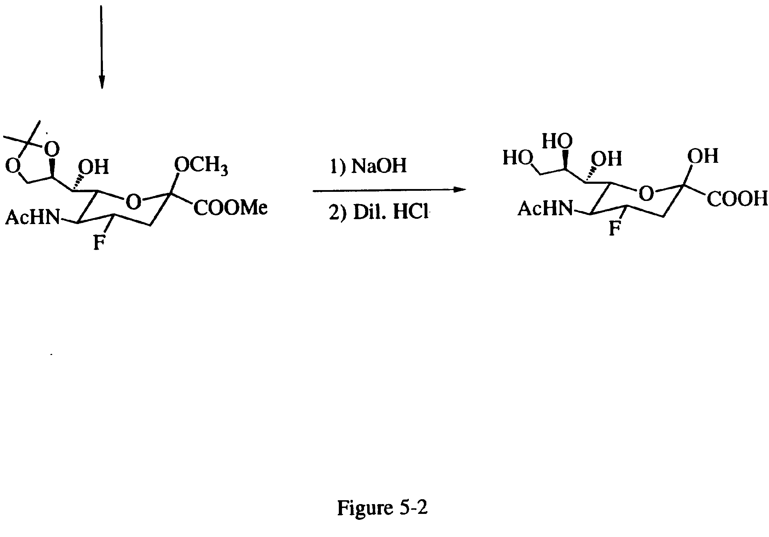

The reaction scheme for this synthesis is shown in Figure 5.

Neu5Ac (500 mg) in 100 ml of methanol and 1.25 g of Dowex-50W (H⁺) are heated under reflux for 20 hr, and treated with decolorizing carbon to afford the methyl ester methyl ketoside of Neu5Ac (Neu5Ac-MEMK). To the ketoside in dry acetone (dried over molecular sieves) is added catalytic p-toluenesulfonic acid monohydrate and the reaction allowed to stir for 4 hr. at room temperature (RT). Neutralization with Dowex®-1 (acetate form) and solvent evaporation gives 8,9-isopropylidene Neu5Ac methyl ester methyl ketoside. This compound is then selectively oxidized to the 4-keto derivative with either pyridinium dichromate (PDC) or ruthenuim tetraoxide, with 4 angstrom molecular sieves in acetonitrile. Mild reduction with borane-ammonia in tetrahydrofuran affords the inverted 4-alcohol which may then be converted to the 4-fluoride (with inversion of configuration) with diethylamino sulfur trifluoride (DAST). Deprotection is accomplished by treatment first with sodium hydroxide followed by treatment with dilute HCI/Dowex-50W (H+) to give 4-fluoro-N-acetylneuraminic acid.

### 6. Synthesis of 2-(4-Chloro-1-Naphthyl)-4-Fluoro Neu5Ac

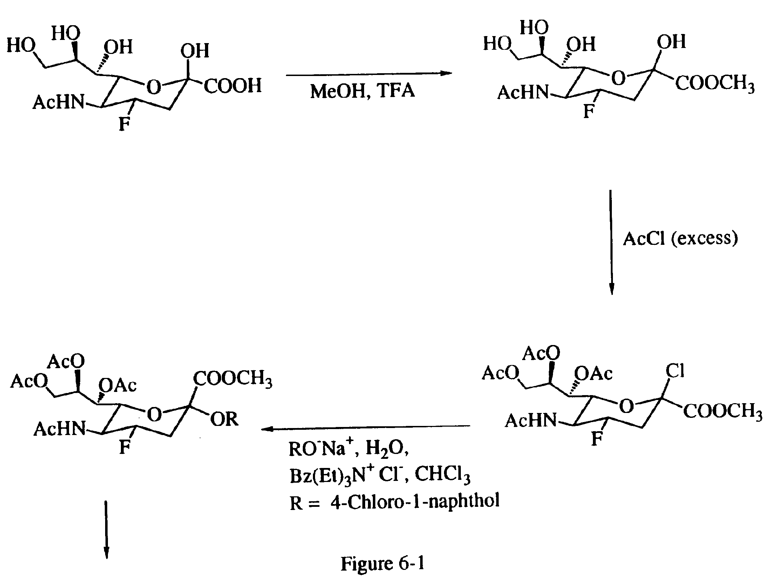

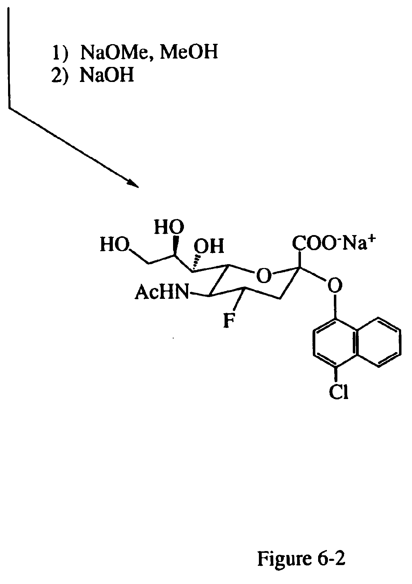

The reaction scheme for this synthesis is shown in Figure 6.

The 4-fluoro Neu5Ac of Example 5 is converted to its methyl ester by treatment in methanol with trifluoroacetic acid Reaction in excess acetyl chloride acetylates the free alcohol groups and effects conversion to the glycosyl chloride simultaneously. The sodium salt of 4-chloro-1-naphthol (4-CN) was prepared with 1 equivalent of NaOH in water. Reaction of the glycosyl chloride in chloroform with this aqueous 4-CN solution (2.5 equivalents) in the presence of benzyl triethylammonium chloride (phase transfer reagent) affords the coupled chromogenic compound. Deprotection of the acetates and methyl ester group is accomplished by treatment with sodium methoxide and sodium hydroxide which affords the sodium salt of 2-(4-chloro-1-naphthyl)-4-fluoro Neu5Ac.

### 7. Enzymatic testinq of 4-Deoxy Neu5Ac

50 µl of an influenza virus solution was mixed with a reaction mixture containing 50 µI of the substrate 4-methylumbelliferyl-Neu5Ac at various concentrations in the submillimolar to millimolar range, 150 µl of the inhibitor 4-deoxy Neu5Ac at various concentrations in the submillimolar to millimolar range, and 50 µl of 100 mM CaCl₂. All solutions were made up in 50 mM sodium acetate buffer, pH 5.9, to a final volume of 500 µl. After incubation at 37°C for 15 to 30 minutes (depending on virus strain), the reaction was terminated by adding 500 µl of 1 M Tris, pH 9.0 with 1.33% Ethanol. The fluorescence intensity was measured at an excitation wavelength of 360 nm and an emission wavelength of 450 nm with a fluorescence spectrophotometer (Hitachi Model 3010). 4-methyl-υmbelliferone in 1 M Tris, pH 9.0 with 1.33% Ethanol served as a standard. Enzyme activity was expressed as mM of Neu5Ac liberated per minute per 50 µl of virus. A plot of 1/v vs. 1/[S] for varying concentrations of substrate and inhibitor showed typical competitive inhibition. Plotting the slopes of the 1/v vs. 1/[S] plot versus the inhibitor concentration allowed for the calculation of Kᵢ for 4-deoxy Neu5Ac as follows:

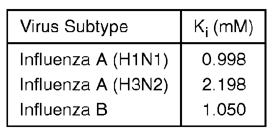

(The native substrate, Neu5Ac, had a Kᵢ=0.626 mM when the Influenza A (H1N1 ) virus was used.)

The Kᵢ for 4-deoxy Neu5Ac indicates how the compound interacts with the enzyme as well as the rate at which it interacts. In general, the lower the Kᵢ, the greater the degree of inhibition at any given substrate and inhibitor concentration. It is also desirable to have a modified compound which can interact with an enzyme in a similar manner as the native compound without compromising its ability as a substrate. The Kᵢ gives a first indication of the compound's interaction with the enzyme.

Modifications of the above-described modes for carrying out the invention that are obvious to those of skill in the fields of organic chemistry virology, biochemistry medical diagnostics, and related fields are intended to be within the scope of the following claims.

## Claims (Claims for the following Contracting State(s) : AT, BE, CH, LI, DE, DK, FR, GB, GR, IT, LU, NL, SE)

1. A method of detecting human influenza neuraminidase activity in a clinical sample suspected of having such activity comprising:
(a) incubating the sample with a chromogenic modified N-acetylneuraminic acid substrate of the formula: 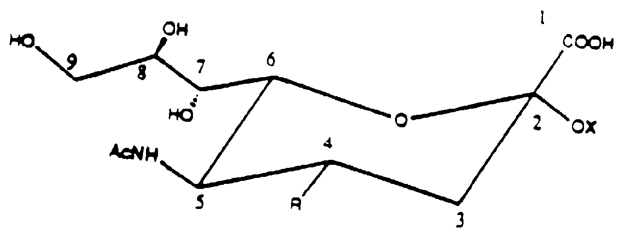 where Ac represents acetyl, R represents hydrogen, fluorine or methoxy and X represents a chromogenic group that exhibits distinct color when cleaved from the substrate or a salt of said substrate; and
(b) detecting neuraminidase activity by observing whether the sample-substrate mixture exhibits said color after step (a).

2. The method of claim 1 wherein the clinical sample is a pharyngeal, nasopharyngeal or respiratory secretion.

3. The method of claim 1 or 2 wherein R represents hydrogen and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl

4. The method of claim 1 or 2 wherein R represents fluorine and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl

5. The method of claim 1 or 2 wherein R represents methoxy and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl and 6-bromo-2-naphthyl.

6. A method of selectively detecting a specific type of human influenza neuraminidase activity in a clinical sample suspected of having human influenza neuraminidase activity comprising:
(a) incubating the sample with a chromogenic modified N-acetylneuraminic acid substrate of the formula: 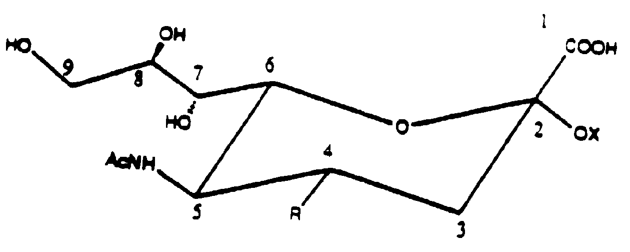 where Ac represents acetyl, R represents hydrogen, fluorine or methoxy, and X represents a chromogenic group that exhibits distinct color when cleaved from the substrate or a salt of said substrate;
(b) observing the color exhibited by the sample-substrate mixture after step (a); and
(c) comparing said color to colors exhibited by activity standards of human influenza neuraminidase of said specific type and other types of human influenza neuraminidase on said substrate.

7. The method of claim 6 wherein the specific type of human influenza neuraminidase activity is human influenza A neuraminidase activity, human influenza B neuraminidase activity, or parainfluenza neuraminidase activity

8. The method of claim 6 or 7 wherein the clinical sample is a pharyngeal, nasopharyngeal, or respiratory secretion.

9. The method of claim 6, 7 or 8 wherein R represents hydrogen and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

10. The method of claim 6, 7 or 8 wherein R represents fluorine and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl

11. The method of claim 6, 7 or 8 wherein R represents methoxy and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl

12. A chromogenic substrate useful for detecting human influenza neuraminidase activity in a clinical sample suspected of having such activity, said substrate having the formula: 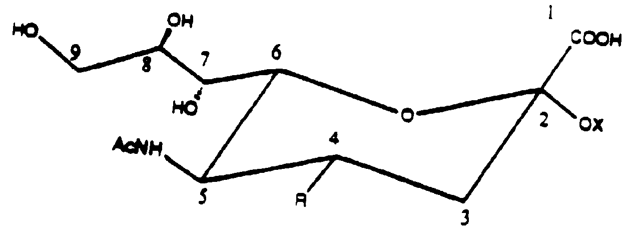 where Ac represents acetyl, R is methoxy or fluorine, and X is a chromogenic group that exhibits a distinct color when cleaved from the substrate and salts of said substrate.

13. The chromogenic substrate of claim 12 wherein R represents methoxy and the chromogenic group is selected from the group consisting of 4-methylumbelliferyl, 3-cyanoumbelliferyl, 2- or 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

14. The chromogenic substrate of claim 12 wherein R represents fluorine and the chromogenic group is selected from the group consisting of 4-methylumbelliferyl, 3-cyanoumbelliferyl, 2- or 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

15. A chromogenic substrate useful for detecting human influenza neuraminidase activity in a clinical sample suspected of having such activity, said substrate having the formula: 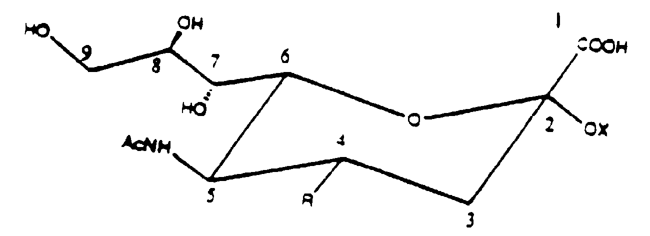 where Ac represents acetyl, R is hydrogen, and X is a chromogenic group selected from the group consisting of 3-cyanoumbelliferyl, 2- or 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl and salts of said substrate.

## Claims (Claims for the following Contracting State(s) : ES)

1. A method of detecting human influenza neuraminidase activity in a clinical sample suspected of having such activity comprising:
(a) incubating the sample with a chromogenic modified N-acetylneuraminic acid substrate of the formula: 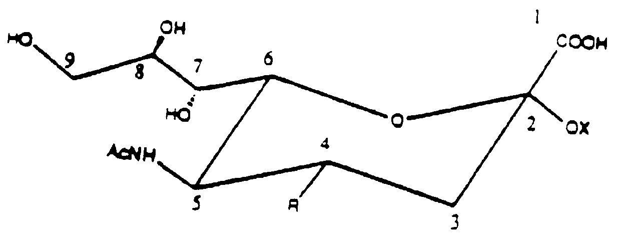 where Ac represents acetyl, R represents hydrogen, fluorine or methoxy, and X represents a chromogenic group that exhibits distinct color when cleaved from the substrate or a salt of said substrate; and
(b) detecting neuraminidase activity by observing whether the sample-substrate mixture exhibits said color after step (a).

2. The method of claim 1 wherein the clinical sample is a pharyngeal, nasopharyngeal or respiratory secretion.

3. The method of claim 1 or 2 wherein R represents hydrogen and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

4. The method of claim 1 or 2 wherein R represents fluorine and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl

5. The method of claim 1 or 2 wherein R represents methoxy and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl and 6-bromo-2-naphthyl.

6. A method of selectively detecting a specific type of human influenza neuraminidase activity in a clinical sample suspected of having human influenza neuraminidase activity comprising:
(a) incubating the sample with a chromogenic modified N-acetylneuraminic acid substrate of the formula: 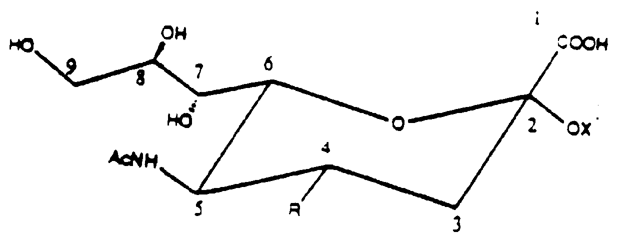 where Ac represents acetyl, R represents hydrogen, fluorine or methoxy and X represents a chromogenic group that exhibits distinct color when cleaved from the substrate or a salt of said substrate;
(b) observing the color exhibited by the sample-substrate mixture after step (a); and
(c) comparing said color to colors exhibited by activity standards of human influenza neuraminidase of said specific type and other types of human influenza neuraminidase on said substrate.

7. The method of claim 6 wherein the specific type of human influenza neuraminidase activity is human influenza A neuraminidase activity, human influenza B neuraminidase activity, or parainfluenza neuraminidase activity.

8. The method of claim 6 or 7 wherein the clinical sample is a pharyngeal, nasopharyngeal, or respiratory secretion.

9. The method of claim 6, 7 or 8 wherein R represents hydrogen and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

10. The method of claim 6, 7 or 8 wherein R represents fluorine and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl

11. The method of claim 6, 7 or 8 wherein R represents methoxy and X is selected from the group consisting of 4-methylumbelliferyl, 3-cyanoumbelliferyl, 2-nitrophenyl, 4-nitrophenyl, 3-resorufin, 5-bromo-4-chloro-3-indolyl, 4-nitrophenylazophenyl, 4-nitrophenylazoresorcinyl, 3-methoxyphenyl 3-dimethylaminophenyl, 4-chloro-1-naphthyl, and 6-bromo-2-naphthyl.

12. A process for the preparation of a chromogenic substrate useful for detecting human influenza neuraminidase activity in a clinical sample suspected of having such activity, said substrate having the formula: 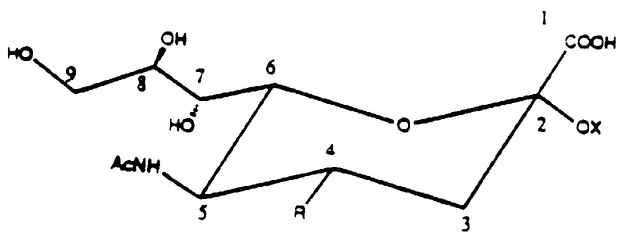 where Ac represents acetyl, R is methoxy or fluorine, and X is a chromogenic group that exhibits a distinct color when cleaved from the substrate and salts of said substrate, which process comprises protecting the functional groups of N-acetylneuraminic acid (Neu5Ac) at the 1,2,7,8, and 9 positions, modifying the 4 position as indicated, deprotecting the 1, 2, 7, 8 and 9 positions, and coupling the 4-modified Neu5Ac with the chromogen.

13. The process of claim 12 wherein R represents methoxy and the chromogenic group is selected from the group consisting of 4-methylumbelliferyl, 3-cyanoumbelliferyl, 2- or 4-nitrophenyi, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

14. The process of claim 12 wherein R represents fluorine and the chromogenic group is selected from the group consisting of 4-methylumbelliferyl, 3-cyanoumbelliferyl, 2- or 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl.

15. A process for the preparation of a chromogenic substrate useful for detecting human influenza neuraminidase activity in a clinical sample suspected of having such activity, said substrate having the formula: 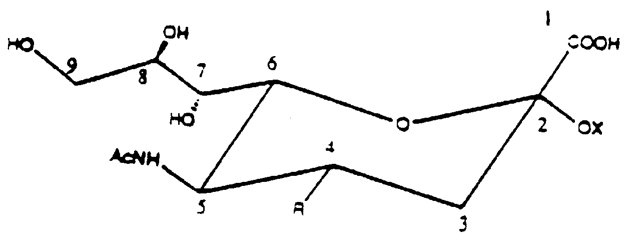 where Ac represents acetyl, R is hydrogen, and X is a chromogenic group selected from the group consisting of 3-cyanoumbelliferyl, 2- or 4-nitrophenyl, 3-resorufin, 5-bromo-4-chloro-3-indolyl, 4-nitrophenylazophenyl, 4-nitrophenylazoresorcinyl, 3-methoxyphenyl, 3-dimethylaminophenyl, 4-chloro-1-naphthyl, and 6-bromo-2-naphthyl and salts of said substrate, which process comprises protecting the functional groups of N-acetylneuraminic acid (Neu5Ac) at the 1, 2, 7, 8, and 9 positions, modifying the 4 position as indicated, deprotecting the 1, 2, 7, 8 and 9 positions, and coupling the 4-modified Neu5Ac with the chromogen.

## Patentansprüche (Patentansprüche für folgende(n) Vertragsstaat(en) : AT, BE, CH, LI, DE, DK, FR, GB, GR, IT, LU, NL, SE)

1. Verfahren zum Nachweis der humanen Influenza-Neuraminidase-Aktivität in einer klinischen Probe, bei der Verdacht auf eine solche Aktivität besteht, umfassend:
(a) das Inkubieren der Probe mit einem chromogen modifizierten N-Acetylneuraminsäure-Substrat der Formel: 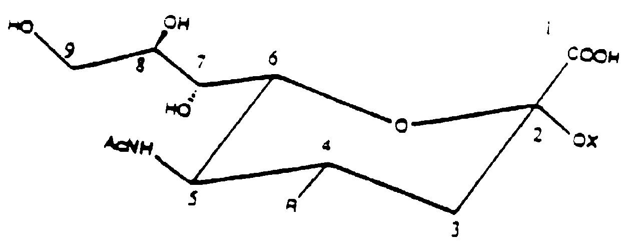 worin Ac für Acetyl steht, R für Wasserstoff, Fluor oder Methoxy steht und X für eine chromogene Gruppe steht, die eine unterschiedliche Farbe zeigt, wenn sie vom Substrat oder einem Salz dieses Substrats abgespalten wird; und
(b) das Nachweisen der Neuraminidase-Aktivität durch Beobachten, ob das Probe-Substrat-Gemisch diese Farbe nach Schritt (a) zeigt.

2. Verfahren nach Anspruch 1, worin die klinische Probe eine pharyngeale, nasopharyngeale oder respiratorische Sekretion ist.

3. Verfahren nach Anspruch 1 oder 2, worin R für Wasserstoff steht und X gewählt ist aus der Gruppe, bestehend aus 4-Methylumbelliferyl. 3-Cyanoumbelliferyl, 2-Nitrophenyl, 4-Nitrophenyl, 3-Resorufin, 5-Brom-4-chlor-3-indo- lyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

4. Verfahren nach Anspruch 1 oder 2, worin R für Fluor steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

5. Verfahren nach Anspruch 1 oder 2, worin R für Methoxy steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

6. Verfahren zum selektiven Nachweis einer spezifischen Art der humanen Influenza-Neuraminidase-Aktivität in einer klinischen Probe, bei der Verdacht auf eine humane Influenza-Neuraminidase-Aktivität besteht, umfassend:
(a) das Inkubieren der Probe mit einem chromogen modifizierten N-Acetylneuraminsäure-Substrat der Formel: 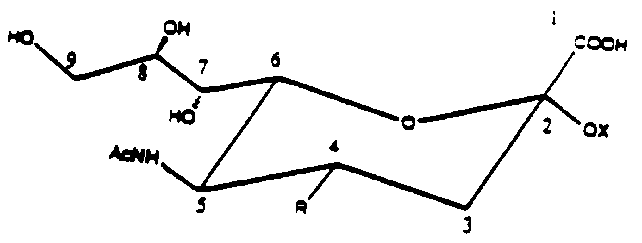 worin Ac für Acetyl steht, R für Wasserstoff, Fluor oder Methoxy steht und X für eine chromogene Gruppe steht, die eine unterschiedliche Farbe zeigt, wenn sie vom Substrat oder einem Salz dieses Substrats abgespalten wird; und
(b) das Beobachten der Farbe, die das Probe-Substrat-Gemisch nach Schritt (a) zeigt; und
(c) das Vergleichen dieser Farbe mit Farben, wie sie von Aktivitäts-Standardwerten der humanen Influenza-Neuraminidase dieser spezifischen Art und anderer Arten der humanen Influenza-Neuraminidase auf diesem Substrat gezeigt werden.

7. Verfahren nach Anspruch 6, worin die spezifische Art der humanen Influenza-Neuraminidase-Aktivität eine humane Influenza-A-Neuraminidase-Aktivität, eine humane Influenza-B-Neuraminidase-Aktivität oder eine Parainfluenza-Neuraminidase-Aktivität ist.

8. Verfahren nach Anspruch 6 oder 7, worin die klinische Probe eine pharyngeale, nasopharyngeale oder respiratorische Sekretion ist.

9. Verfahren nach Anspruch 6, 7 oder 8, worin R für Wasserstoff steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indo- lyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

10. Verfahren nach Anspruch 6, 7 oder 8, worin R für Fluor steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

11. Verfahren nach Anspruch 6, 7 oder 8, worin R für Methoxy steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

12. Chromogenes Substrat, das nützlich ist zum Nachweis der humanen Influenza-Neuraminidaso-Aktivilät in einer klinischen Probe, bei der Verdacht auf eine solche Aktivität besteht, wobei dieses Substrat die Formel aufweist: 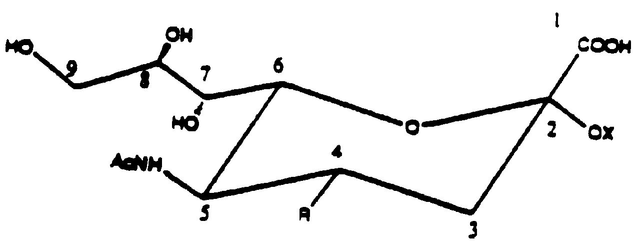 worin Ac für Acetyl steht, R für Methoxy oder Fluor steht und X eine chromogene Gruppe ist, die eine unterschiedliche Farbe zeigt, wenn sie vom Substrat und Salzen dieses Substrats abgespalten wird.

13. Chromogenes Substrat nach Anspruch 12, worin R für Methoxy steht und die chromogene Gruppe gewählt ist aus der Gruppe, bestehend aus 4-Methylumbelliferyl, 3-Cyanoumbelliferyl, 2- oder4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

14. Chromogenes Substrat nach Anspruch 12, worin R für Fluor steht und die chromogene Gruppe gewählt ist aus der Gruppe, bestehend aus 4-Methylumbelliferyl, 3-Cyanoumbelliferyl, 2- oder4-Nitrophenyl. 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

15. Chromogenes Substrat, das nützlich ist zum Nachweis der humanen Influenza-Neuraminidase-Aktivität in einer klinischen Probe, bei der Verdacht auf eine solche Aktivität besteht, wobei dieses Substrat die Formel aufweist: 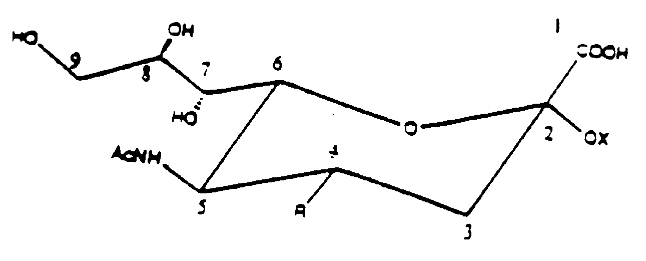 worin Ac für Acetyl steht, R Wasserstoff ist und X eine chromogene Gruppe ist, gewählt aus der Gruppe, bestehend aus 3-Cyanoumbelliferyl, 2- oder 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl und Salzen dieses Substrats.

## Patentansprüche (Patentansprüche für folgende(n) Vertragsstaat(en) : ES)

1. Verfahren zum Nachweis der humanen Influenza-Neuraminidase-Aktivität in einer klinischen Probe, bei der Verdacht auf eine solche Aktivität besteht, umfassend:
(a) das Inkubieren der Probe mit einem chromogen modifizierten N-Acetylneuraminsäure-Substrat der Formel: 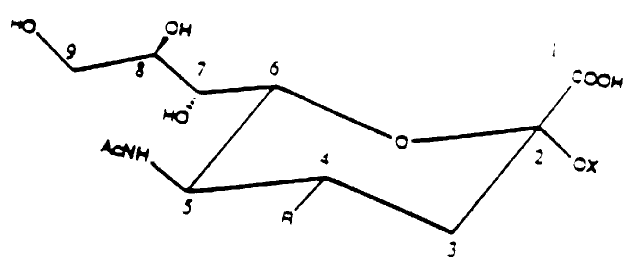 worin Ac für Acetyl steht, R für Wasserstoff, Fluor oder Methoxy steht und X für eine chromogene Gruppe steht, die eine unterschiedliche Farbe zeigt, wenn sie vom Substrat oder einem Salz dieses Substrats abgespalten wird; und
(b) das Nachweisen der Neuraminidase-Aktivität durch Beobachten, ob das Probe-Substrat-Gemisch diese Farbe nach Schritt (a) zeigt.

2. Verfahren nach Anspruch 1, worin die klinische Probe eine pharyngeale, nasopharyngeale oder respiratorische Sekretion ist.

3. Verfahren nach Anspruch 1 oder 2, worin R für Wasserstoff steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indo- lyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

4. Verfahren nach Anspruch 1 oder 2, worin R für Fluor steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

5. Verfahren nach Anspruch 1 oder 2, worin R für Methoxy steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

6. Verfahren zum selektiven Nachweis einer spezifischen Art der humanen Influenza-Neuraminidase-Aktivität in einer klinischen Probe, bei der Verdacht auf eine humane Influenza-Neuraminidase-Aktivität besteht, umfassend:
(a) das Inkubieren der Probe mit einem chromogen modifizierten N-Acetylneuraminsäure-Substrat der Formel: 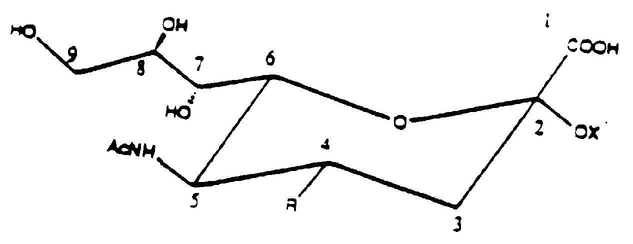 worin Ac für Acetyl steht, R für Wasserstoff, Fluor oder Methoxy steht und X für eine chromogene Gruppe steht, die eine unterschiedliche Farbe zeigt, wenn sie vom Substrat oder einem Salz dieses Substrats abgespalten wird;
(b) das Beobachten der Farbe, die das Probe-Substrat-Gemisch nach Schritt (a) zeigt: und
(c) das Vergleichen dieser Farbe mit Farben, wie sie von Aktivitäts-Standardwerten der humanen Influenza-Neuraminidase dieser spezifischen Art und anderer Arten der humanen Influenza-Neuraminidase auf diesem Substrat gezeigt werden.

7. Verfahren nach Anspruch 6, worin die spezifische Art der humanen Influenza-Neuraminidase-Aktivität eine humane Influenza-A-Neuraminidase-Aktivität, eine humane Influenza-B-Neuraminidase-Aktivität oder eine Parainfluenza-NeuraminidaseAktivität ist.

8. Verfahren nach Anspruch 6 oder 7, worin die klinische Probe eine pharyngeale, nasopharyngeale oder respiratorische Sekretion ist.

9. Verfahren nach Anspruch 6, 7 oder 8, worin R für Wasserstoff steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indo- lyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

10. Verfahren nach Anspruch 6, 7 oder 8, worin R für Fluor steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

11. Verfahren nach Anspruch 6, 7 oder 8, worin R für Methoxy steht und X gewählt ist aus der Gruppe, bestehend aus 4-Methylumbelliferyl, 3-Cyanoumbelliferyl, 2-Nitrophenyl, 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

12. Verfahren zur Herstellung eines chromogenen Substrats, das nützlich ist zum Nachweis der humanen Influenza-Neuraminidase-Aktivität in einer klinischen Probe, bei der Verdacht auf eine solche Aktivität besteht, wobei dieses Substrat die Formel aufweist: 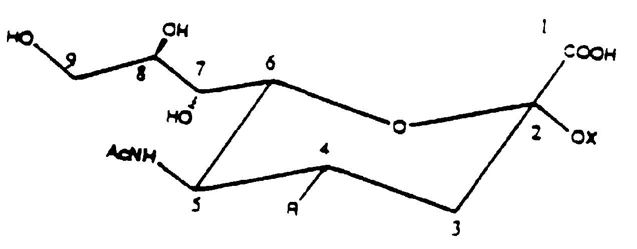 worin Ac für Acetyl steht, R für Methoxy oder Fluor steht und X eine chromogene Gruppe ist, die eine unterschiedliche Farbe zeigt, wenn sie vom Substrat und Salzen dieses Substrats abgespalten wird, welches Verfahren den Schutz der funktionellen Gruppen der N-Acetylneuraminsäure (Neu5Ac) an den 1-, 2-, 7-, 8- und 9-Positionen, die Modifikation der 4-Position wie vorgegeben, die Schutzentfernung an den 1-, 2-, 7-, 8- und 9-Positionen und die Kopplung des 4-modifizierten Neu5Ac mit dem Chromogen umfaßt

13. Verfahren nach Anspruch 12, worin R für Methoxy steht und die chromogene Gruppe gewählt ist aus der Gruppe, bestehend aus 4-Mothylumbelliferyl, 3-Cyanoumbelliferyl, 2- oder 4-Nitrophenyi, 3-Resorufin, 5-Brom-4-chlor-3-in- dolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

14. Verfahren nach Anspruch 12, worin R für Fluor steht und die chromogene Gruppe gewählt ist aus der Gruppe, bestehend aus 4-Methylumbelliferyl, 3-Cyanoumbelliferyl, 2-oder 4-Nitrophenyl, 3-Resorufin, 5-Brom-4-chlor-3-in- dolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl.

15. Verfahren zur Herstellung eines chromogenen Substrats, das nützlich ist zum Nachweis der humanen Influenza-Neuraminidase-Aktivität in einer klinischen Probe, bei der Verdacht auf eine solche Aktivität besteht, wobei dieses Substrat die Formel aufweist: 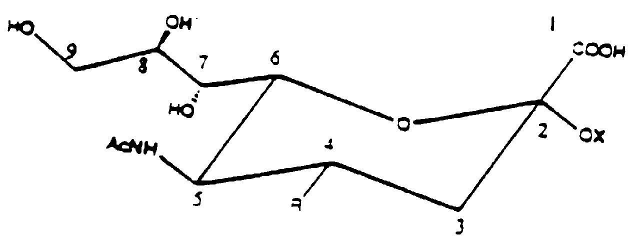 worin Ac für Acetyl steht, R Wasserstoff ist und X eine chromogene Gruppe ist, gewählt aus der Gruppe, bestehend aus 3-Cyanoumbelliferyl, 2- oder 4-Nitrophenyl, 3-Resorufin, 5-Brom-4-chlor-3-indolyl, 4-Nitrophenylazophenyl, 4-Nitrophenylazoresorcinyl, 3-Methoxyphenyl, 3-Dimethylaminophenyl, 4-Chlor-1-naphthyl und 6-Brom-2-naphthyl und Salzen dieses Substrats, welches Verfahren den Schutz der funktionellen Gruppen der N-Acetylneuraminsäure (Neu5Ac) an den 1-, 2-, 7-, 8- und 9-Positionen, die Modifikation der 4-Position wie vorgegeben, die Schutzentfernung an den 1-, 2-, 7-, 8- und 9-Positionen und die Kopplung des 4-modifizierten Neu5Ac mit dem Chromogen umfaßt.

## Revendications (Revendications pour l'(les) Etat(s) contractant(s) suivant(s) : suivants : AT, BE, CH, LI, DE, DK, FR, GB, GR, IT, LU, NL, SE)

1. Procédé de détection de l'activité de neuraminidase de la grippe humaine, dans un échantillon clinique susceptible de présenter une telle activité, comprenant les étapes consistant à :
(a) incuber l'échantillon en présence d'un substrat acide N-acétylneuraminique modifié, chromogène, de formule : 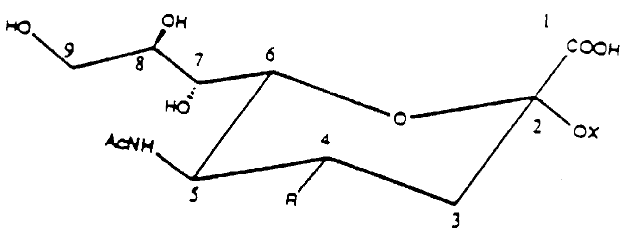 dans laquelle Ac représente un groupe acétyle, R représente un atome d'hydrogène, un atome de fluor ou un groupe méthoxy et X représente un groupe chromogène qui présente une couleur distincte lorsqu'il est coupé du substrat ou d'un sel dudit substrat ; et
(b) détecter l'activité de neuraminidase en observant si le mélange échantillon-substrat présente ladite couleur après l'étape (a).

2. Procédé selon la revendication 1, dans lequel l'échantillon clinique est une sécrétion pharyngienne, nasopharyn- gienne ou respiratoire.

3. Procédé selon la revendication 1 ou 2, dans lequel R représente un atome d'hydrogène et X est choisi dans le groupe constitué par les radicaux 4-méthylumbelliféryle, 3-cyanoumbelliféryle, 2-nitrophényle, 4-nitrophényle, 3-résorufine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

4. Procédé selon la revendication 1 ou 2, dans lequel R représente un atome de fluor et X est choisi dans le groupe constitué par les radicaux 4-méthylumbelliféryle, 3-cyanoumbelliféryle, 2-nitrophényle, 4-nitrophényle, 3-résorufime, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

5. Procédé selon la revendication 1 ou 2, dans lequel R représente un groupe méthoxy et X est choisi dans le groupe constitué par les radicaux 4-méthylumbelliféryle, 3-cyanoumbelliféryle, 2-nitrophényle, 4-nitrophényle, 3-résorufine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

6. Procédé permettant de détecter sélectivement un type spécifique d'activité de neuraminidase de la grippe humaine, dans un échantillon clinique susceptible de présenter une activité de neuraminidase de la grippe humaine, comprenant les étapes consistant à :
(a) incuber l'échantillon en présence d'un substrat acide N-acétylneuraminique modifié, chromogène, de formule : 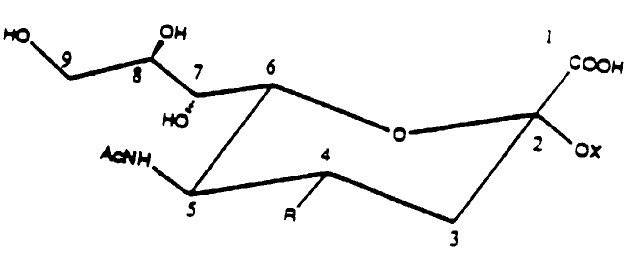 dans laquelle Ac représente un groupe acétyle, R représente un atome d'hydrogène, un atome de fluor ou un groupe méthoxy et X représente un groupe chromogène présentant une couleur distincte lorsqu'il est coupé du substrat ou d'un sel dudit substrat :
(b) observer la couleur présentée par le mélange échantillon-substrat après l'étape (a) ; et
(c) comparer ladite couleur aux couleurs présentées par des étalons d'activité de neuraminidase de la grippe humaine dudit type spécifique et d'autres types de neuraminidase de la grippe humaine sur ledit substrat.

7. Procédé selon la revendication 6, dans lequel le type spécifique d'activité de neuraminidase de la grippe humaine est l'activité de neuraminidase de la grippe humaine A, l'activité de neuraminidase de la grippe humaine B ou l'activité de neuraminidase de la parainfluenza.

8. Procédé selon la revendication 6 ou 7, dans lequel l'échantillon clinique est une sécrétion pharyngienne, naso- pharyngienne ou respiratoire.

9. Procédé selon la revendication 6, 7 ou 8, dans lequel R représente un atome d'hydrogène et X est choisi dans le groupe constitué par les radicaux 4-méthylumbelliféryle, 3-cyanoumbelliféryle, 2-nitrophényle, 4-nitrophényle, 3-résorufi ine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

10. Procédé selon la revendication 6, 7 ou 8, dans lequel R représente un atome de fluor et X est choisi dans le groupe constitué par les radicaux 4-méthylumbelliféryle, 3-cyanoumbelliféryle, 2-nitrophényle, 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

11. Procédé selon la revendication 6, 7 ou 8, dans lequel R représente un groupe méthoxy et X est choisi dans le groupe constitué par les radicaux 4-méthylumbelliféryle, 3-cyanoumbelliféryle, 2-nitrophényle, 4-nitrophényle, 3-résorufime, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

12. Substrat chromogène utilisable pour détecter l'activité de neuraminidase de la grippe humaine, dans un échantillon clinique susceptible de présenter une telle activité, ledit substrat ayant la formule 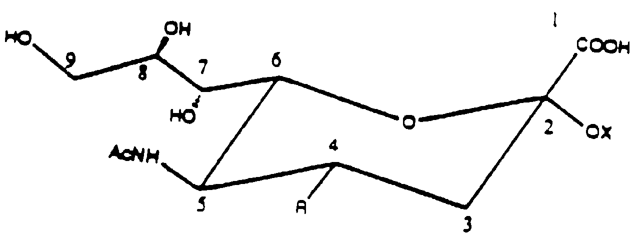 dans laquelle Ac représente un groupe acétyle, R représente un groupe méthoxy ou un atome de fluor et X est un groupe chromogène présentant une couleur distincte lorsqu'il est coupé du substrat et les sels dudit substrat.

13. Substrat chromogène selon la revendication 12, dans lequel R représente un groupe méthoxy et dans lequel le groupe chromogène est choisi dans le groupe constitué par les radicaux 4-méthylumbelliféryle, 3-cyanoumbelliféryle, 2- ou 4-nitrophényle, 3-résorufine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

14. Substrat chromogène selon la revendication 12, dans lequel R représente un atome de fluor et dans lequel le groupe chromogène est choisi dans le groupe constitué par les radicaux 4-méthylumbelliféryle, 3-cyanoumbelliféryle, 2- ou 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

15. Substrat chromogène utilisable pour détecter l'activité de neuraminidase de la grippe humaine, dans un échantillon clinique susceptible de présenter une telle activité, ledit substrat ayant la formule 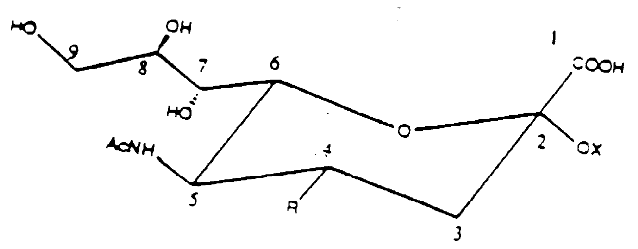 dans laquelle Ac représente un groupe acétyle, R est un atome d'hydrogène et X est un groupe chromogène choisi dans le groupe constitué par les radicaux 3-cyanoumbelliféryle, 2- ou 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle et les sels dudit substrat.

## Revendications (Revendications pour l'(les) Etat(s) contractant(s) suivant(s) : suivant : ES)

1. Procédé de détection de l'activité de neuraminidase de la grippe humaine, dans un échantillon clinique susceptible de présenter une telle activité, comprenant les étapes consistant à :
(a) incuber l'échantillon en présence d'un substrat acide N-acétylneuraminique modifié, chromogène, de formule : 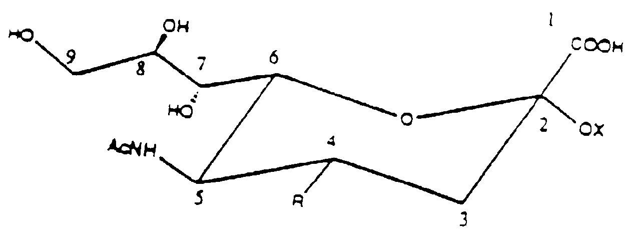 dans laquelle Ac représente un groupe acétyle, R représente un atome d'hydrogène, un atome de fluor ou un groupe méthoxy et X représente un groupe chromogène qui présente une couleur distincte lorsqu'il est coupé du substrat ou d'un sel dudit substrat : et
(b) détecter l'activité de neuraminidase en observant si le mélange échantillon-substrat présente ladite couleur après l'étape (a).

2. Procédé selon la revendication 1, dans lequel l'échantillon clinique est une sécrétion pharyngienne, nasopharyn- gienne ou respiratoire.

3. Procédé selon la revendication 1 ou 2, dans lequel R représente un atome d'hydrogène et X est choisi dans le groupe constitué par les radicaux 4-méthylumbelliféryle, 3-cyanoumbelliféryle, 2-nitrophényle, 4-nitrophényle, 3-résorufi ine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

4. Procédé selon la revendication 1 ou 2, dans lequel R représente un atome de fluor et X est choisi dans le groupe constitué par les radicaux 4-méthylumbelliféryle, 3-cyanoumbelliféryle, 2-nitrophényle, 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

5. Procédé selon la revendication 1 ou 2, dans lequel R représente un groupe méthoxy et X est choisi dans le groupe constitué par les radicaux 4-méthylumbelliféryle, 3-cyanoumbelliféryle, 2-nitrophényle, 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

6. Procédé permettant de détecter sélectivement un type spécifique d'activité de neuraminidase de la grippe humaine, dans un échantillon clinique susceptible de présenter une activité de neuraminidase de la grippe humaine, comprenant les étapes consistant à :
(a) incuber l'échantillon en présence d'un substrat acide N-acétylneuraminique modifié, chromogène, de formule : 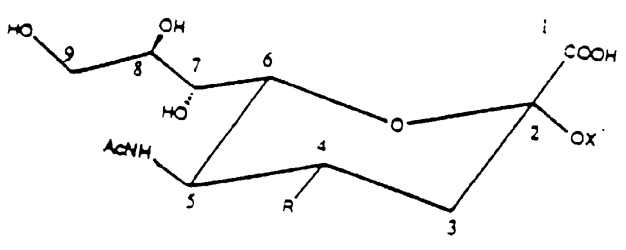 dans laquelle Ac représente un groupe acétyle, R représente un atome d'hydrogène, un atome de fluor ou un groupe méthoxy et X représente un groupe chromogène présentant une couleur distincte lorsqu'il est coupé du substrat ou d'un sel dudit substrat :
(b) observer la couleur présentée par le mélange échantillon-substrat après l'étape (a) ; et
(c) comparer ladite couleur aux couleurs présentées par des étalons d'activité de neuraminidase de la grippe humaine dudit type spécifique et d'autres types de neuraminidase de la grippe humaine sur ledit substrat.

7. Procédé selon la revendication 6, dans lequel le type spécifique d'activité de neuraminidase de la grippe humaine est l'activité de neuraminidase de la grippe humaine A, l'activité de neuraminidase de la grippe humaine B ou l'activité de neuraminidase de la parainfluenza.

8. Procédé selon la revendication 6 ou 7, dans lequel l'échantillon clinique est une sécrétion pharyngienne, naso- pharyngienne ou respiratoire.

9. Procédé selon la revendication 6, 7 ou 8, dans lequel R représente un atome d'hydrogène et X est choisi dans le groupe constitué par les radicaux 4-méthylumbelliféryle, 3-cyanoumbelliféryle, 2-nitrophényle, 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

10. Procédé selon la revendication 6, 7 ou 8, dans lequel R représente un atome de fluor et X est choisi dans le groupe constitué par les radicaux 4-méthylumbelliféryle, 3-cyanoumbelliféryle, 2-nitrophényle, 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

11. Procédé selon la revendication 6, 7 ou 8, dans lequel R représente un groupe méthoxy et X est choisi dans le groupe constitué par les radicaux 4-méthylumbelliféryle, 3-cyanoumbelliféryle, 2-nitrophényle, 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

12. Procédé de préparation d'un substrat chromogène utilisable pour détecter l'activité de neuraminidase de la grippe humaine, dans un échantillon clinique susceptible de présenter une telle activité, ledit substrat ayant la formule: 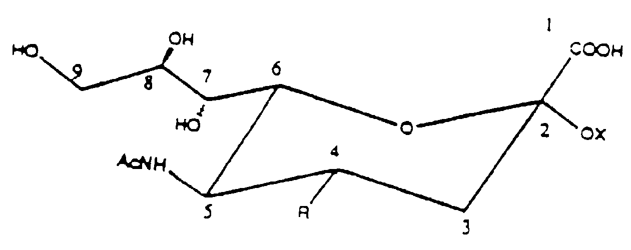 dans laquelle Ac représente un groupe acétyle, R représente un groupe méthoxy ou un atome de fluor et X est un groupe chromogène présentant une couleur distincte lorsqu'il est coupé du substrat et les sels dudit substrat, lequel procédé comprend les étapes consistant à protéger les groupes fonctionnels de l'acide N-acétylneuraminique (Neu5Ac) aux positions 1, 2, 7, 8 et 9, à modifier la position 4 comme indiqué, à déprotéger les positions 1, 2, 7, 8 et 9 et à coupler le Neu5Ac modifié en position 4 avec le groupe chromogène.

13. Procédé selon la revendication 12, dans lequel R représente un groupe méthoxy et le groupe chromogène est choisi dans le groupe constitué par les radicaux 4-méthylumbelliféryle, 3-cyanoumbelliféryle, 2- ou 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle.

14. Procédé selon la revendication 12, dans lequel R représente un atome de fluor et le groupe chromogène est choisi dans le groupe constitué par les radicaux 4-méthylumbelliféryle, 3-cyanoumbelliféryle, 2- ou 4-nitrophényle, 3-résorufime, 5-bromo-4-chloro-3-indolyle, 4-nitrophenylazophényle, 4-nitrophénylazorésorcinyle, 3-méthoxyphényle, 3-diméthylaminophényle, 4-chloro-1-naphtyle et 6-bromo-2-naphtyle.

15. Procédé de préparation d'un substrat chromogène utilisable pour détecter l'activité de neuraminidase de la grippe humaine, dans un échantillon clinique susceptible de présenter une telle activité, ledit substrat ayant la formule : 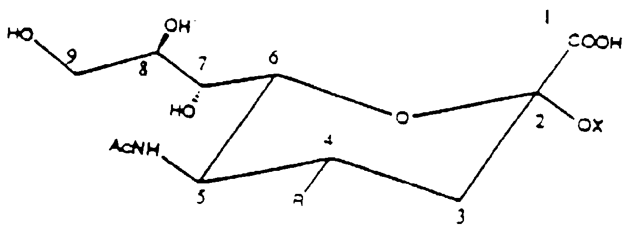 dans laquelle Ac représente un groupe acétyle, R est un atome d'hydrogène et X est un groupe chromogène choisi dans le groupe constitué par les radicaux 3-cyanoumbelliféryle, 2- ou 4-nitrophényle, 3-résorufiine, 5-bromo-4-chloro-3-indolyle, 4-nitrophénylazophényle, 4-nitrophénylazorésorcinyle, 3-méthoxyphényle, 3-diméthylaminophényle, 4-chloro-1-naphtyle et 6-bromo-2-naphtyle et les sels dudit substrat, lequel procédé comprend les étapes consistant à protéger les groupes fonctionnels de l'acide N-acétylneuraminique (Neu5Ac) aux positions 1, 2, 7, 8 et 9, à modifier la position 4 comme indiqué, à déprotéger les positions 1, 2, 7, 8 et 9 et à coupler le Neu5Ac modifié en position 4 avec le groupe chromogène.
